# EUROPÄISCHE PATENTANMELDUNG

(11) **EP 1 698 608 A1**
(43) Veröffentlichungstag der Anmeldung: **06.09.2006**
(21) Anmeldenummer: 06003096.2
(22) Anmeldetag: 16.02.2006
(51) Int. Cl.: C07C 29/10, C07C 31/20

(54) **Verfahren zur Herstellung von Alkylenglykolen**

(30) Priorität: 01.03.2005 DE 102005009133
(71) Anmelder: Clariant Produkte (Deutschland) GmbH, 65929 Frankfurt am Main (DE)
(72) Erfinder: Stankowiak, Achim, Dr., 84503 Altötting (DE); Holzhauser, Erwin, 84140 Ganghofen (DE); Snell, Alexander, Dr., 84503 Altötting (DE)
(74) Vertreter: Mikulecky, Klaus

(57) **Zusammenfassung**

Gegenstand der Erfindung ist ein Verfahren zur Herstellung von Alkylenglykolen durch Hydratisierung von Alkylenoxiden in Gegenwart von Polyalkylenglykoldialkylethern der Formel

R¹-O-[-(CH₂CH₂O)ₘ(CH(CH₃)CH₂)-O]ₙR²

worin
m = 0 - 100,
n = 0 - 100,
wobei n+m mindestens gleich 1 ist,
R¹ ein C₁- bis C₆-Alkylrest,
R² ein C₁- bis C₆-Alkylrest, wobei R² von R¹ verschieden sein kann, bedeuten,
mit der Maßgabe, dass bei mindestens 50 mol-% des Polyalkylenglykoldialkylethers m+n größer oder gleich 11 ist.

## Beschreibung

Die vorliegende Erfindung betrifft ein Verfahren zur Herstellung von Alkylenglykolen durch Hydrolyse der entsprechenden Alkylenoxide in Gegenwart eines Polyglykoldialkylethers.

Nach dem Stand der Technik ist bekannt, dass Alkylenoxide zu den entsprechenden Alkylenglykolen hydrolysiert werden können (Ullmann's Encyclopedia of Industrial Chemistry, Wiley-VCH Verlag, 6. Auflage, CD-ROM 2003). Ein Nachteil der bekannten Verfahren besteht darin, dass ein sehr hoher Wasserüberschuss (das Molekularverhältnis von Alkylenoxid zu Wasser beträgt von 1 : 6 bis 1: 20) erforderlich ist, um die Bildung von Di-, Tri- und Polyalkylenglykolen möglichst zu vermeiden. Die anfallende wässrige Rohalkylenglykol-Lösung wird in Verdampfern aufkonzentriert und in mehreren Vakuumkolonnen fraktioniert destilliert. Dies ist mit erheblichem Apparate- und Energieaufwand und somit hohen Kosten verbunden. Außerdem beträgt die Selektivität trotz des hohen Wasserüberschusses beispielsweise bei der Herstellung von Monoethylenglykol nur ca. 90%. Daneben entstehen ca. 9 % Diglykol sowie 1 % Triglykol und höhere Ethylenglykole (siehe: K. Weissermel, H. J. Arpe "Industrielle Organische Chemie", 5. Aufl., 1998, Seite 167-168). In der Literatur sind eine Vielzahl von Verfahren beschrieben worden, die die gewünschte Selektivität erhöhen, beziehungsweise die erforderlichen Wassermengen reduzieren.

DE-A-29 24 680 beschreibt ein Verfahren zur Alkylenglykolherstellung, bei dem die katalytische Hydrolyse in Gegenwart von CO₂ über ein Glykolester-Zwischenprodukt und in Anwesenheit eines organischen Lösemittels, durchgeführt wird. Als Lösungsmittel werden Ester, Ketone und Ether beschrieben; insbesondere Aceton und Dioxan. Bei dem beschriebenen Prozess werden sehr hohe Monoethylenglykol-Selektivitäten von bis zu 99 % erreicht, allerdings unter Einsatz von extrem hohen Katalysatormengen (0,22 Mol Katalysator pro Liter Ethylenoxid), die am Wirkungsgrad dieses Verfahrens zweifeln lassen. Nachteilig bei diesen Verfahren wirkt sich ferner aus, dass eine mit erhöhtem apparativem Aufwand verbundene Zugabe von unter Druck stehendem Kohlendioxid erfolgen muss.

US-4760200 beschreibt ein Verfahren, bei dem die Hydrolyse in Gegenwart von einem organischen Co-Lösungsmittel, vorzugsweise 1,2-Dimethoxyethan, und ggf. wasserlöslichen Metallat-Anionen der VI. Nebengruppe des Periodensystems durchgeführt wird. Die Monoethylenglykol-Selektivitäten sind gut, allerdings ist das bevorzugte Lösungsmittel 1,2-Dimethoxyethan giftig und kann sowohl die Fortpflanzungsfähigkeit als auch das Kind im Mutterleib schädigen.

Ein ebenfalls Metallat-katalysierter Prozess wird in EP-A-0156448 beschrieben. Hierbei kommen Benzol, Xylol, Toluol, Dichlormethan oder 1,1,2-Trichlorethan als Co-Lösemittel zum Einsatz, um den verwendeten Katalysator zu recyclieren.

Ziel der vorliegenden Erfindung ist die Beseitigung der genannten Nachteile. Der Erfindung wurde die Aufgabe zugrunde gelegt, ein Verfahren zur Herstellung von Alkylenglykolen zu entwickeln, welches es ermöglicht, den Prozess ohne oder mit nur geringem Wasserüberschuss durchzuführen, bei gleich bleibender oder sogar erhöhter Selektivität bezüglich der Bildung von Monoalkylenglykolen.

Gegenstand der Erfindung ist ein Verfahren zur Herstellung von Alkylenglykolen durch Hydratisierung von Alkylenoxiden in Gegenwart von Polyalkylenglykoldialkylethern der Formel

R¹-O-[-(CH₂CH₂O)ₘ(CH(CH₃)CH₂)-O]ₙ-R²

worin
m = 0 - 100,
n = 0 - 100,
wobei n+m mindestens gleich 1 ist,
R¹ ein C₁- bis C₆-Alkylrest,
R² ein C₁- bis C₆-Alkylrest, wobei R² von R¹ verschieden sein kann, bedeuten, mit der Maßgabe, dass bei mindestens 50 mol-% des Polyalkylenglykoldialkylethers m+n größer oder gleich 11 ist.

R¹ bedeutet vorzugsweise Methyl oder Ethyl.

R² bedeutet vorzugsweise Methyl oder Ethyl.

m bedeutet vorzugsweise 4 bis 60, besonders bevorzugt 11 bis 30.

n bedeutet vorzugsweise 1 bis 20.

m+n ist bei mindestens 50 mol-% des Polyalkylenglykoldialkylethers vorzugsweise größer als 12, insbesondere größer als 13, speziell größer als 14.

Die Alkylenoxide sind vorzugsweise Ethylen-, Propylen- oder Butylenoxid, oder deren Mischungen.

Die Menge in der der Polyalkylenglykoldialkylether zugegeben werden kann, beträgt etwa das 0,1-20fache (Gew.-%) bezogen auf die eingesetzte Menge Wasser; vorzugsweise das 1 bis 10fache (Gew.-%). Die relative Molmasse kann zwischen 400 und 12000 g/mol betragen; vorzugsweise 500 bis 4000 g/mol.

Die Polyalkylenglykoldialkylether sind an sich bekannt oder können nach bekannten Verfahren durch Umsetzung von Polyalkylenglykolen mit einem Alkylierungsmittel hergestellt werden.

Ein Katalysator ist nicht zwingend erforderlich, kann jedoch verwendet werden, um die Selektivität weiter zu erhöhen. Als Katalysatoren eignen sich basische Verbindungen, wie beispielsweise Alkali- und Erdalkalisalze. Zu diesen Katalysatoren zählen Kalium- und Natriumhydroxid, Kalium- und Natriumacetat, Kalium- und Natriumphosphat, Kalium- und Natriumhalogenide, Kalium- und Natriumcarbonate und der gleichen. Der Katalysator kann als Salz zugegeben werden oder *in situ* gebildet werden.

Das erfindungsgemäße Verfahren kann kontinuierlich oder diskontinuierlich durchgeführt werden. Bevorzugt wird eine kontinuierliche Fahrweise. Darüber hinaus erfolgt das Verfahren unter Temperatur- und Druckverhältnissen wie sie bei technischen Verfahren üblich sind (Ullmann's Encyclopedia of Industrial Chemistry, Wiley-VCH Verlag, 6. Auflage, CD-ROM 2003). Bevorzugt werden Temperaturen zwischen 80 und 400°C und einem Druck unter 50 bar. Besonders bevorzugt wird eine Temperatur zwischen 100 und 300°C und ein Druck zwischen 20 und 40 bar. Der Prozess kann unter CO₂-Atmosphäre durchgeführtwerden und verläuft dann vermutlich über eine Carbonat-Zwischenstufe.

Das erfindungsgemäße Verfahren wird nun in einigen Beispielen noch näher erläutert:

### Beispiele

Als Reaktor wurde ein Rührautoklav aus rostfreiem Stahl und von 500 ccm Inhalt verwendet. Der Autoklav war ausgestattet mit Gaseinleitungsrohr, thermoelektrischen Elementen, Rührer, elektrischem Heizmantel und Kühlschlange. Während des Betriebs wurde der Reaktor mit einer Mischung aus destilliertem Wasser, ggf. 1 % Kaliumiodid als Katalysator, 200 g Polyethylenglykoldimethylether mit einem Molgewicht von 540 g/mol (Polyglykol DME 500 der Firma Clariant mit einem Anteil an Homologen mit n≥11 Von durchschnittlich 58 % (bestimmt mittels Gaschromatographie)) und entweder N₂ oder CO₂ beschickt und auf die Reaktionstemperatur erhitzt. Nach Erreichen der gewünschten Reaktionstemperatur wurde der Reaktor mit Ethylenoxid beschickt. Nach 2 Stunden Reaktionszeit wurde der Reaktor entspannt und das Reaktionsprodukt gaschromatographisch analysiert.

In den Vergleichsbeispielen kamen je 200g 1,2-Dimethoxyethan (Monoethylenglykoldimethylether der Firma Clariant mit n=1), Tetraethylenglykoldimethylether (Firma Clariant, n=4) oder Polyglykol DME 250 (Polyethylenglykoldimethylether mit einem Molgewicht von ca. 240 g/mol und einem Anteil an Homologen mit n≥11 von maximal 5% (bestimmt mittels Gaschromatographie)) zum Einsatz.

Die folgende Tabelle zeigt die verwendeten Reaktionsbedingungen und das hergestellte Monoethylenglykol (MEG), Diethylenglykol (DEG) und Triethylenglykol (TEG) in Gew.-%. Die Umsetzung von Ethylenoxid zu Glykolen war nahezu quantitativ.

**Tabelle 1**

| Nr. | Temp. [°C] | EO [g] | H₂O [g] | Verhältnis EO zu H₂O | Polyalkylenglykoldiether | Atmosphäre | Kat. | MEG | DEG | TEG |
|---|---|---|---|---|---|---|---|---|---|---|
| 1^{a)} | 160 | 20,0 | 72,0 | 1:3,6 | - | N₂ | - | 75,3 | 21,2 | 3,5 |
| 2^{a)} | 160 | 20,0 | 100 | 1 : 5 | - | N₂ | - | 84,6 | 14,1 | 1,3 |
| 3^{a)} | 160 | 20,0 | 200 | 1:10 | - | N₂ | - | 91,4 | 8,0 | 0,6 |
| 4^{b)} | 160 | 20,0 | 40,0 | 1 : 2 | Dimethoxyethan | N₂ | Na₂MoO₄ | 87,4 | 11,2 | 1,4 |
| 5^{b)} | 160 | 20,0 | 80,0 | 1 : 4 | Dimethoxyethan | N₂ | Na₂MoO₄ | 88,6 | 9,3 | 2,1 |
| 6^{b)} | 160 | 20,0 | 46,0 | 1:2,3 | Tetraethylenglykoldimethylether | N₂ | | 85,3 | 12,8 | 1,9 |
| 7^{b)} | 160 | 20,0 | 46,0 | 1 : 2,3 | Polyglykol DME 250 | N₂ | - | 85,3 | 12,8 | 1,9 |
| 8 | 160 | 20,0 | 72,0 | 1:3,6 | Polyglykol DME 500 | N₂ | - | 97,5 | 2,5 | 0,0 |
| 9 | 160 | 20,0 | 46,0 | 1:2,3 | Polyglykol DME 500 | N₂ | - | 94,0 | 5,4 | 0,6 |
| 10 | 200 | 20,0 | 72,0 | 1:3,6 | Polyglykol DME 500 | CO₂ | Kl | 96,2^{c)} | 1,6 | 0,0 |
| 11 | 160 | 20,0 | 46,0 | 1:2,3 | Polyglykol DME 500 | N₂ | Kl | 96,6 | 2,0 | 1,4 |

| | | | | | | | | | | |
|---|---|---|---|---|---|---|---|---|---|---|
| ^{a)} Vergleichsbeispiele ohne Polyglykol DME 500 | | | | | | | | | | |
| ^{b)} Vergleichsbeispiele gem. US 4760200 | | | | | | | | | | |
| ^{c)} Es konnten 2,2% Ethylencarbonat im Reaktionsprodukt nachgewiesen werden. | | | | | | | | | | |

Die Beispiele machen deutlich, dass durch Zugabe von Polyglykol DME 500 eine höhere Selektivität als bei Zusatz von erheblichen Mengen Wasser erreicht werden kann. Der erforderliche Energiebedarf zur Abtrennung der Wassermengen vom Endprodukt ist wesentlich höher als der zur Abtrennung des Polyalkylenglykoldimethylethers. Somit ist das erfindungsgemäße Verfahren wesentlich wirtschaftlicher.

## Patentansprüche

1. Verfahren zur Herstellung von Alkylenglykolen durch Hydratisierung von Alkylenoxiden in Gegenwart von Polyalkylenglykoldialkylethern der Formel
R¹-O-[-(CH₂CH₂O)ₘ(CH(CH₃)CH₂)-O]ₙ-R²
worin
m = 0 - 100,
n=0-100,
wobei n+m mindestens gleich 1 ist,
R¹ ein C₁- bis C₆-Alkylrest,
R² ein C₁- bis C₆-Alkylrest, wobei R² von R¹ verschieden sein kann, bedeuten,
mit der Maßgabe, dass bei mindestens 50 mol-% des Polyalkylenglykoldialkylethers m+n größer oder gleich 11 ist.

2. Verfahren nach Anspruch 1, worin R¹ Methyl oder Ethyl bedeutet.

3. Verfahren nach Anspruch 1 und/oder 2, worin R² Methyl oder Ethyl bedeutet.

4. Verfahren nach einem oder mehreren der Ansprüche 1 bis 3, worin m 4-60 bedeutet.

5. Verfahren nach einem oder mehreren der Ansprüche 1 bis 4, worin n 1-20 bedeutet.

6. Verfahren nach einem oder mehreren der Ansprüche 1 bis 5, worin m+n bei mindestens 50 mol-% des Polyalkylenglykoldialkylethers größer als 12 ist

7. Verfahren nach einem oder mehreren der Ansprüche 1 bis 6, worin die Alkylenoxide Ethylen-, Propylen- oder Butylenoxid, oder deren Mischungen sind.

8. Verfahren nach einem oder mehreren der Ansprüche 1 bis 7, worin die Menge der zugegebenen Polyalkylenglykoldialkylether 1 bis 20 Gew.-% bezogen auf die eingesetzte Menge Alkylenoxid beträgt.
